(19) **Europäisches Patentamt / European Patent Office / Office européen des brevets**

(11) **EP 1 663 168 B1**

(12) # EUROPEAN PATENT SPECIFICATION

(45) Date of publication and mention
of the grant of the patent:
**11.02.2009 Bulletin 2009/07**

(21) Application number: **04769203.3**

(22) Date of filing: **27.08.2004**

(51) Int Cl.:
***A61K 9/22*** *(2006.01)*    ***A61K 9/70*** *(2006.01)*
***A61K 9/68*** *(2006.01)*

(86) International application number:
**PCT/IB2004/002787**

(87) International publication number:
**WO 2005/023227 (17.03.2005 Gazette 2005/11)**

(54) **NICOTINE FORMULATIONS AND USE THEREOF**

NIKOTINFORMULIERUNGEN UND IHRE VERWENDUNG

FORMULATIONS DE NICOTINE ET LEURS UTILISATIONS

(84) Designated Contracting States:
**AT BE BG CH CY CZ DE DK EE ES FI FR GB GR HU IE IT LI LU MC NL PL PT RO SE SI SK TR**

(30) Priority: **08.09.2003 SE 0302387**
**08.09.2003 US 501061 P**

(43) Date of publication of application:
**07.06.2006 Bulletin 2006/23**

(73) Proprietor: **McNeil AB**
**25109 Helsingborg (SE)**

(72) Inventors:
• **EK, Bo, Ragnar**
**S-114 53 Stockholm (SE)**
• **MIHRANYAN, Albert**
**0021 Yerevan (AM)**
• **ANDERSSON, Sven-Börje**
**S-112 87 Stockholm (SE)**
• **BOSSON, Bengt, Ake**
**S-112 87 Stockholm (SE)**

• **LINDBERG, Nils-Olof**
**S-216 15 Malmö (SE)**

(74) Representative: **Hedenström, John Allan et al**
**Pfizer Health AB**
**Patent Department**
**P.O. Box 941**
**251 09 Helsingborg (SE)**

(56) References cited:
**WO-A1-20/04064811    WO-A2-20/04056363**
**DE-A1- 1 517 264    GB-A- 1 362 611**
**US-A- 4 735 218    US-A- 5 795 979**

• **DATABASE CA [Online] CHEMICAL ABSTRACTS SERVICE, COLUMBUS, OHIO, US; TAKANASHI, SEIZI: "Process for producing granular algal adsorbents" XP002316182 retrieved from STN Database accession no. 1988:97074 & CN 85 104 064 CN (C. S. KOGYO K. K., JAPAN) 28 January 1987 (1987-01-28)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

## Technical Field

The present invention pertains to pharmaceutical products for delivering nicotine, where nicotine is absorbed in and/or adsorbed onto cellulose of non-seed organism origin, preferably with large surface area. The nicotine-containing cellulose has a high content of stabile nicotine in a bioavailable form that is well suited for several pharmaceutical preparations. The invention also comprises methods for manufacturing of a nicotine-containing cellulose matrix. Also included are methods and systems for delivering nicotine to a subject as well as use and manufacture of said pharmaceutical preparations.

## Background of the Invention

Tobacco dependence and reduction thereof

In recent years, with the recognition of the harmful effects of tobacco smoking, there have been numerous campaigns and programs by governmental agencies and various health groups and other interested organisations to disseminate information about the adverse health effects resulting from tobacco smoking. Moreover, and as a result of this recognition of the harmful effects, there have been many programs directed to reducing smoking incidence.

Nicotine is an organic compound and is the principal alkaloid of tobacco. Nicotine is the main addictive ingredient in the tobacco used in cigarettes, cigars, snuff and other nicotine-containing products. Nicotine is also an addictive drug, though, and smokers characteristically display a strong tendency to relapse after having successfully abstained from smoking for a time. Nicotine is the world's second most used drug, after caffeine from coffee and tea.

The main problem with tobacco smoking is its enormous implications on health. Today it is estimated that smoking related diseases cause some 3 - 4 million deaths per year. In the US Surgeon General's 1988 report on The Health Consequences of Smoking, it was estimated that in the US alone about 300.000 deaths are caused each year by diseases related to cigarette smoking. In fact, excessive smoking is now recognised as one of the major health problems throughout the world. This grim consequence of tobacco smoking has urged many medical associations and health authorities to take very strong actions against the use of tobacco.

Even though tobacco smoking is decreasing in many developed countries today it is hard to see how the societies could get rid of the worlds second most used drug.

The best thing a heavy smoker can do is to reduce smoking and preferably stop smoking completely. Experience shows, however, that most smokers find this extremely difficult since, mostly, tobacco smoking results in a dependence disorder or craving. WHO has in its International Classification of Disorders a diagnosis called Tobacco Dependence. Other organizations, like the American Psychiatric Association call the addiction Nicotine Dependence. It is generally accepted that the difficulties in withdrawing from smoking result from the fact that smokers are dependent on nicotine. The most important risk factors are substances that are formed during the combustion of tobacco, such as carbon monoxide, tar products, aldehydes, and hydrocyanic acid.

Effects of nicotine

The administration of nicotine can provide satisfaction. The usual administration method is by smoking, by using e g a cigarette, a cigar or a pipe. As smoking has health hazards it is desirable to formulate alternative means of administering nicotine in a pleasurable manner that can be used to facilitate withdrawal from smoking and/or used as a replacement for smoking.

When smoking a cigarette, nicotine is quickly absorbed into the smoker's blood and reaches the brain around ten seconds after inhalation. The quick uptake of nicotine gives the consumer a rapid satisfaction, or kick. The satisfaction lasts during the smoking time of the cigarette and for a period of time thereafter. The poisonous, toxic, carcinogenic, and addictive nature of smoking has initiated efforts to search for methods, compositions and devices, which would help in breaking the habit of smoking cigarettes.

Nicotine is an addictive poisonous alkaloid $C_{10}H_{14}N_2$, derived from the tobacco plant. Nicotine is also used as an insecticide.

Nicotine replacement products

One way to reduce smoking is to provide nicotine in a form or manner other than by smoking. Some products have been developed to fulfil this need. Nicotine containing formulations are currently the dominating means for treating tobacco dependence.

The success rates in achieving reduction in the incidence of smoking have been relatively low using presently known products. The present state of the art involves both behavioural approaches and pharmacological approaches. More than 80 % of the tobacco smokers who quit smoking after using some behavioural or pharmacological approach to reduce smoking incidence generally relapse and return to the habit of smoking at their former rate of smoking within about a one year's period of time.

As an aid for those who are willing to stop smoking there are several ways and forms of nicotine replacement products available on the market, such as nicotine chewing gum and nicotine transdermal patch.

Several methods and means have been described for diminishing the desire of a subject to use tobacco, which comprises the step of administering to the subject nicotine or a derivative thereof as described in e.g. US 5,810,018 (oral nicotine spray), US 5,939,100 (nicotine-containing microspheres) and US 4,967,773 (nicotine-containing lozenge).

Nicotine-containing nose drops have been reported (Russell et al., British Medical Journal, Vol. 286, p. 683 (1983); Jarvis et al., Brit. J. of Addiction, Vol. 82, p. 983 (1987)). Nose drops, however, are difficult to administer and are not convenient for use at work or in other public situations. Administrating nicotine by way of delivering nicotine directly into the nasal cavity by spraying is known from US 4,579.858, DE 32 41 437 and WO/93 127 64. There may, though, be local nasal irritation with use of nasal nicotine formulations. The difficulty in administration also results in unpredictability of the size of the dose of nicotine administered.

The use of skin patches for transdermal administration of nicotine has been reported (Rose, in Pharmacologic Treatment of Tobacco Dependence, (1986) pp. 158-166, Harvard Univ. Press). Nicotine-containing skin patches that are in wide use today can cause local irritation and the absorption of nicotine is slow and affected by cutaneous blood flow.

Also, inhaling devices resembling a cigarette are known for uptake of nicotine vapours as suggested in US 5,167,242.

An aerosol for deposing nicotine in the lungs is disclosed in DE 3 241 437.

Mouth sprays comprising nicotine are known in the art, e g according to US 6,024,097 wherein is disclosed a method of assisting a smoker in giving up the smoking habit whereby is used a plurality of aerosol dispensers comprising progressively lesser concentrations of nicotine. The aerosol is intended to be administered into the mouth. The liquid in the dispensers essentially consists of nicotine and alcohol.

US 2002/0059939 discloses a device for administering nicotine by sucking nicotine-containing granules into the mouth. The nicotine, only suggested in the form of nicotine particles, may be incorporated in spheres consisting inter alia of sugar, starch and cellulose. The only example relates to nicotine salt adhered to sugar spheres. It is to be understood that the cellulose mentioned is normal cellulose, i e cellulose from wood.

US 5,254,346 discloses a transdermal patch, which may contain nicotine and which may contain a surfactant comprising methylcellulose.

DE 1 517 264 discloses a tobacco substitute comprising inter alia nicotine and combustible cellulose from wood. The substitute is intended to release nicotine upon burning as with tobacco smoking.

<u>Nicotine dosage forms</u>

One of the most successful approaches to date in reducing the incidence of smoking relies upon nicotine-containing dosage forms, e g chewing gum, transdermal patch, nasal spray, inhaler, sublingual tablet that are designed to reduce smoking withdrawal symptoms. The reported success rate is approximately twice that of other previously used methods.

<u>Prior art and problems thereof</u>

There is a need to provide pharmaceutical formulations of nicotine wherein the nicotine is stabilized against degradation while at the same being easily accessible for the user without the nicotine release being obtained through burning, as with tobacco. The above need is not entirely achieved through nicotine formulations known in the art. The present invention achieves this need through use of cellulose of non-seed organism origin as a carrier for nicotine.

Products that are used as smoking substitutes and/or as smoking cessation aids based on nicotine can contain nicotine in many different forms. The nicotine form is selected from the group consisting of the free base form of nicotine, a nicotine salt, a nicotine derivative, such as a nicotine cation exchanger, such as polyacrylate with free carboxylic acid groups, a nicotine inclusion complex or nicotine in any non-covalent binding, nicotine bound to starch microspheres, and mixtures thereof.

The most preferable embodiment incorporates nicotine in its free base form or as a water-soluble pharmaceutically acceptable salt, either *per se* or adsorbed on an adsorbent, or as a complex with a cation exchanger or mixtures of the foregoing, as an inclusion complex, such as a cyclodextrin complex, e g β-cyclodextrin. Any other suitable pharmaceutically acceptable form of nicotine may also be employed.

Numerous nicotine salts are known, primarily those of the acids according to below Table 1.

**Table 1.** Possible acids for nicotine salt formation

| Acid | Molar ratio* of Acid:nicotine |
|---|---|
| Formic | 2:1 |
| Acetic | 3:1 |
| Propionic | 3:1 |
| Butyric | 3:1 |
| 2-Methylbutyric | 3:1 |
| 3-Methylbutyric | 3:1 |
| Valeric | 3:1 |
| Lauric | 3:1 |
| Palmitic | 3:1 |
| Tartaric | 2:1 |
| Citric | 2:1 |
| Malic | 2:1 |
| Oxalic | 2:1 |
| Benzoic | 1:1 |
| Gentisic | 1:1 |
| Gallic | 1:1 |
| Phenylacetic | 3:1 |
| Salicylic | 1:1 |
| Phthalic | 1:1 |
| Picric | 2:1 |
| Sulfosalicylic | 1:1 |
| Tannic | 1:5 |
| Pectic | 1:3 |
| Alginic | 1:2 |
| Hydrochloric | 2:1 |
| Chloroplatinic | 1:1 |
| Silicotungstic | 1:1 |
| Pyruvic | 2:1 |
| Glutamic | 1:1 |
| Aspartic | 1:1 |

*recommended upon manufacturing

Nicotine salts preferred in the present invention are tartrate, hydrogen tartrate, hydrochloride, acetate and salicylate, although salts of the other acids of Table 1 are not excluded.

The traditional solid nicotine species as e g nicotine resin complex, nicotine β-cyclodextrin complex, and salts as e g nicotine bitartrate dihydrate are all chemically stabile with low volatility of nicotine and are used in dosage forms such as gums and tablets.

Some nicotine forms, which are not included in well-known pharmacopoeias, will have to be chemically and physically characterized as well as toxicologically evaluated prior to being used as active pharmaceutical ingredients.

By the present invention high concentration of liquid free nicotine base, the readily bioavailable form, or other liquid, semisolid or solid forms of nicotine are transferred to chemically stabile, solid products from which the release of nicotine may be immediate.

Nicotine absorbed in and/or adsorbed onto cellulose

The present invention comprises a pharmaceutical product comprising a cellulose carrier for administration of nicotine. The nicotine-cellulose matrix comprises cellulose with a high capacity for binding of drugs and other chemicals, and to which nicotine can be bound reversibly. The cellulose has a non-seed organism origin. Nicotine absorbed in and/or adsorbed to highly porous cellulose of non-seed organism origin, e g cellulose from algae, has not been disclosed. The cellulose is readily wetted with water and saliva, but is not soluble in said liquids, and it can therefore be regarded as an inert carrier. The nicotine-cellulose matrix preferably has a high pore volume and a good flowability.

Cellulose is useful as a nicotine carrier for several reasons. Cellulose is white, tasteless, stabile and biocompatible. It exhibits good compactability in drug mixtures. In its microcrystalline form cellulose is extensively used as an excipient in many tablet compositions. Microcrystalline cellulose is a purified and partly depolymerised cellulose prepared by treating alpha cellulose, obtained as pulp from fibrous plant material, with mineral acid. Annually about 10.000-30.000 tons of microcrystalline cellulose is used in the manufacturing of pharmaceutical tablets.

There are at least four ways to obtain cellulose material. The industrially most important way involves isolation of cellulose fibres from seed organisms in a separation processes to remove lignin and hemicelluloses. About 1/3 of dry wood is alpha cellulose. Such cellulose material is used for production of microcrystalline cellulose. Another way consists in the biosynthesis of cellulose by algae, bacteria and fungi.

The cellulose of the present invention has a non-seed organism origin. One preferred cellulose carrier in the present invention is cellulose from Cladophora species, an alga having a unique capacity for binding nicotine by reversible absorption and/or adsorption mechanisms. Cellulose particles from Cladophora species have a highly porous, weblike structure in contrast to cellulose from seed organisms. Nicotine is absorbed into and/or adsorbed onto the sponge-like structure of Cladophora species cellulose and is thereby transferred into stabile form. Nicotine is a labile compound which, when stored unprotected, easily undergoes losses due to evaporation and/or oxidative degradation. The unique structure and the exceptionally large surface area of non-seed organism cellulose imply that a carrier made thereof can protect nicotine in a suitable way. By absorption into the porous structure of such cellulose nicotine is prevented from direct contact with air and consequently stabilized to oxidation.

Nicotine is an amine compound with a pKa-value in aqueous solutions close to 8.0. Noncharged nicotine dominates above pH=8.0 and it is important to note that only the noncharged form of the nicotine molecule can penetrate rapidly through oral or nasal membranes or skin.

Cellulose of algal as well as of fungal or bacterial origin differs from non-seed organism cellulose. The fine structure for algal, fungal and bacterial cellulose is weblike with high crystallinity and large surface area, in the order of 100 $m^2$/g, in contrast to the solid fibres of seed organism cellulose that is characterised by a moderate crystallinity with low porosity and a surface area of less than 1 $m^2$/g. The cellulose of the present invention preferably has a surface area of more than 5 $m^2$/g. Further, it is preferred that particles of the cellulose have a pore volume above 0,01 $cm^3$/g.

The surface area of Cladophora cellulose powder is high, see below Table 2, and is close to the surface area of commercially used adsorbents, that is in the order of about 100-1000 $m^2$/g. See Chemical Engineers' Handbook, Theory of sorption J.H. Perry, C.H. Chilton and S.D. Kirkpatrick (Eds) 14th Ed McGrawHill Book Company New York 1963 16-4.

By absorption into cellulose of non-seed organism origin an amount of nicotine being large in relation to the amount of cellulose can be transferred into solid and chemically stabile form. This stabile solid form is advantageous in connection with production of many dosage forms, such as chewing gums and tablets. The release of nicotine from cellulose of non-seed organism origin may be immediate.

The cellulose particles are loaded with nicotine eg by being mixed with an alcoholic solution of nicotine and evaporation of the alcohol, or via direct absorption from nicotine-saturated air or inert gas. The amount of nicotine loaded via evaporation surpasses the loading from nicotine-saturated air.

The so formed nicotine-cellulose matrix is a white to grey powder. The system can be coloured or flavoured with suitable flavours and sweeteners as desired. In addition, the matrix can also contain buffers, antioxidants and/or preservatives. An added buffer will be released together with nicotine and neutralize or increase the pH of the surrounding solution.

Main advantages obtained with a nicotine-cellulose matrix are:

- Nicotine prevails in a solid and chemically stabile form
- Nicotine prevails in a readily bioavailable and unionised base form
- Reversible binding of nicotine
- Rapid release of nicotine
- Large ratio of nicotine bound to cellulose possible
- Cellulose is odourless and tasteless
- Cellulose is non-toxic and non-irritant
- Cellulose is not absorbed systemically

## Summary of the Invention

The present invention provides for a method for absorption of nicotine into and/or adsorption of nicotine onto cellulose of non-seed organism origin. Thereby nicotine is stabilised. The release of nicotine in aqueous phase from the nicotine-cellulose mixture may be immediate.

Furthermore, the present invention provides for a system for delivering nicotine being absorbed in and/or adsorbed onto non-seed organism cellulose according to claim 1 from suitable dosage forms, such as chewing gums, mouth sprays, nasal sprays, inhaling devices, tablets, such as sublingual tablets, lozenges, buccal sachets, transdermal patches and powders.

## Detailed Description of the Invention

### 1. Definitions

The terms "buccal" and "buccally" and equivalents are herein intended to pertain to all of or any part of the tissue of the oral cavity.

The term "crystallinity" and equivalents are defined according to the following method:

An X-ray diffractometer with Bragg-Brentano geometry was used (Diffraktometer D5000, Siemens, Germany) to get the X-ray spectra. The CuKα radiation was utilised (λ=1.54 Å) and the angle 2θ was set between 10° and 45°. The crystallinity index was calculated as

$$CrI = \frac{I_{002} - I_{am}}{I_{002}} \qquad (1)$$

where $I_{002}$ is the overall intensity of the peak at 2θ about 22° and $I_{am}$ is the intensity of the baseline at 2θ about 18°. See Segal, L., Creely, J. J., Martin, A. E. Jr., and Conrad, C. M., 1959. An empirical method for estimating the degree of crystallinity of native cellulose using the X-ray diffractometer, Textile Res. J., October, 786-794.

The term "surface area" and equivalents are defined according to the following method:

The specific surface area of the powders was obtained from a BET analysis of $N_2$ adsorption isotherms (ASAP 2010, Micrometrics, USA). From the same set of measurements, the total pore volume of the powders was obtained by the ASAP 2010 V4 software. The weight of the samples in these measurements was chosen so as to produce a total surface of 5-10 $m^2$. A BET analysis was also employed to analyse water absorption. The surface area available for water adsorption was obtained based on the principles described by Brunauer, B., Emmett, P.H., and Teller, E., 1938.

The term "alpha-cellulose" and equivalents are defined as per Ward K Occurrence of cellulose. In Cellulose and Cellulose Derivatives E Ott (Ed) Interscience Publishers Inc New York 1943, p 11:

"Alpha-cellulose refers to that portion of industrial cellulose pulps which is insoluble in cold sodium hydroxide of mercerising strength (17.5 or 18%). Beta-cellulose is soluble in such a solution but is precipitated on acidification, while gamma-cellulose remains in solution on acidification."

### 2. Cellulose of non-seed organism origin

The present invention pertains to nicotine bound to cellulose of non-seed organism origin. It is believed that the highly crystalline porous web like structure thereof is the important parameter. Such cellulose is e g cellulose secreted by algae, bacteria and fungi. It is not possible to manufacture cellulose with similar characteristics by simply spray drying a well-ground seed organism cellulose suspension with high surface area. The cellulose will agglomerate during drying and give essentially nonporous particles. Even if the cellulose porosity is preserved during drying by physico-chemical methods the structure is instabile and collapses in moist environment. A drastic decrease in surface area has been found when such cellulose is exposed to humid environment. See Matsumoto K., Nakai Y., Yamemochi E., Oguchi T. and Yamamoto K., Effect of pore size on the gaseous adsorption of ethenzamid on porous crystalline cellulose and the physicochemical stability of ethenzamide after storage. Chem. Pharm. Bull. 46 (1998) 314-318.

The purification procedure is in the same order as the one necessary for seed organism based cellulose. See further Ek R, Gustafsson C, Nutt A, Iversen T and Nyström C. Cellulose powder from Cladophora species algae. J. Mol. Recog. 11 (1998) 263-265. An indication of what that means for industrial production can be obtained by looking at the huge plant for pulp and paper production. Red, brown and green algae contain alpha cellulose in the cell walls. Some typical values are listed in below Table 2. Green algae have the highest amount, 20-40 %, of alpha cellulose in the cell wall. Brown algae and red algae contain up to about 20 % alpha cellulose. See Kerger DR, Cell Walls, in The physiology and biochemistry of algae. RA Lewin (Ed.), Academic press, 1962, New York.

**Table 2.** Typical cell wall contents of alpha cellulose in some algae

| Algae species | Contents of alpha cellulose in % |
|---|---|
| Green algae | |
| Chaetomorpha melagonium | 41 |
| Chladophora rupestris | 28.5 |
| Enteromorpha species | 21 |
| Ulva lactuca | 19 |
| Brown algae | |
| Halidrys siliquosa | 14 |
| Himanthalia lorea | 8 |
| Pelvetia canaliculata | 1.5 |
| Laminaria digitata | 20 |
| Red algae | |
| Griffithsia flusculosa | 22 |
| Porphyra species. | 3.5 |
| Ptilota plumose | 24 |
| Rhodymenia palmate | 7 |

Algae is a raw material for alginate. Alginate is manufactured by harvesting brown algae and by subsequent alkali extraction. In the extraction alginate follows the alkali liquid. The residue in the filtration consists of insoluble parts from the cell walls. The alpha cellulose can be obtained by purifying the filtrate residue. The amount and type of cellulose material in the filter cake depends on the algae, see above Table 1.

Algal cellulose useful in the present invention is cellulose preferably, but not exclusively, obtained from the species in Table 2 as well as from one or more algae selected from

blue-green algae (Cyanophyta), such as Anabaena and Nostoc punctiformae;

green algae (Chlorophyta), such as Chladophora glomerata, Oocystis, e g solitaria and apiculata, Valonia, e g ventricosa, and Chara corallina;

gold algae (Chrysophyta), such as Vaucheria;

dinoflagellates (Pyrrophyta), such as Crypthecodinium cohnii, Gonyaulax polyedra, Scrippsiella hexapraecingula, Dinobryon and Peridinium;

brown algae (Phaeophyta), such as Lessonia nigrescens, Macrocystis pyrifera, Ascophyllum nodosum and Fucus serratus and red algae (Rhodophyta), such as Erythrocladia subintegra.

An advantage of bacterial cellulose, e g secreted by Acetobacter xylinum, is that it can be obtained free from lignin, pectin and hemicelluloses as well as other biogenic products, which are associated with plant cellulose. See Klemm D, Schumann D, Udhardt U and Marsch S, Bacterial synthesized cellulose - artificial blood vessels for microsurgery. Prog.polym.Sci. 26 (2001) 1561-1603.

Cellulose of bacterial origin useful in the present invention is cellulose obtained from bacteria selected from Acetobacter, such as xylinum, pasteurianus, aceti and acetigenus;

Achromobacter;

Aerobacter;

Agrobacterium, such Agrobacterium tumefaciens;

Alcaligenes;

Polyspondylium pallidum;

Pseudomonas;

Rhizobium, such as Rhizobium leguminosarum;

Sarcina;

Sphaerophilus natans;

Xanthomonas campestris, and

Zoogloega.

Cellulose from fungi useful in the present invention is cellulose obtained from fungi selected from Achlya bisexualis;

Colletotrichum lindemuthianum;

Dictyostelium, such as Dictyostelium discoideum;
Microdochium nivale;
Ophiostoma ulmi;
Phytophtora, such as parasitica var. nicotianae and cactorum;
Pythium, such as aphanidermatum, butleri and ultimatum
Saprolegnia, such as parasitica and monoica;

3. The nicotine-cellulose matrix

Amounts of nicotine being up to approximately 50 % of the total mixture weight can be absorbed into and/or adsorbed onto cellulose of non-seed organism origin, while the mixture still remains a surface dry powder.

4. Inhaler

Inhaler, inhalation device or equivalent is intended to mean a reservoir loaded with nicotine allowing air to pass through, whereby nicotine in gaseous form becomes deliverable to a subject.
Cellulose of non-seed organism origin has a high capacity of binding nicotine and reversibly releases it in its gaseous form. By compressing a cellulose tablet or cellulose disc, loaded with nicotine, and adjusting the flow resistance to within the range 0.2-0.6 kPa at a flow rate of 1000 ml/min a suitable matrix for use in an inhalation device can be obtained. When the user puffs on such an inhaling device nicotine is released to the air sucked through the device. The amount of nicotine delivered to the subject in each puff depends on the flow resistance. An average amount is 8-10 $\mu$g nicotine/puff. The uptake of the nicotine mainly takes place in the buccal area.

5. Transdermal patch

Transdermal patch or equivalent is intended to mean a patch with an adhesive layer, which affixes the patch to the skin and which transports a known quantity of a drug to a known area of the skin for a known period of time. A well-known type of a transdermal drug delivery system is of the type "drug-in-adhesive matrix", which is characterized by a three-layer configuration composed of a backing layer, a drug-adhesive layer and a release liner. The drug-adhesive layer is made of a polymeric material in which the drug is dispersed. Also other transdermal patch designs are known in the art.
The time for release of nicotine from the transdermal patch is controlled by the diffusion of drug from the polymer and from the nicotine-cellulose matrix.

6. Tablet

A tablet according to the present invention comprises any lozenge, sublingual tablet, tablet or capsule formulation that delivers nicotine from a nicotine-cellulose matrix to the buccal cavity.
Preferred embodiments contain nicotine in an amount of 0.5-6 mg per tablet calculated as free base/unit dose.

7. Chewing gum

A chewing gum product according to the present invention may be a medicated chewing gum. Medicated chewing gums are herein intended to mean solid or semi-solid, single-dose preparations with a base consisting mainly of gum that are intended to be chewed but not swallowed, whereby the chewing gum acts as a drug delivery system. Such gums contain one or more active substances, which are released upon chewing. After dissolution or dispersion of the active substance in the saliva systemic delivery of the drug takes place through transmucosal uptake throughout the oral cavity.
Preferred embodiments may contain nicotine in an amount of 0.5 - 6 mg calculated as the amount of free base of nicotine per piece chewing gum product.

8. Buccal sachet

A buccal sachet is a drug device with a design similar to that of a small teabag. This delivery system can be described as a portion-in-packet pouch with a strictly measured dose of nicotine. The nicotine-cellulose matrix containing sachet should preferably be placed under the lip, in the same way as snuff users apply tobacco sachets. The sachet should not be dissolved or negatively affected by saliva. At the same time it must allow free penetration of saliva and water, as well as permit extraction of nicotine and other mobile components of the formulation. The material can be made of woven or non-woven fibres. The material can typically be, but is not limited to, heat-sealable teabag paper or non-woven viscose fibre fabric. The systemic dose of nicotine delivered from the sachet should preferably be in the same range as the nicotine dose that oral snuff users and chewing tobacco users typically get. In the literature is mentioned that users of tobacco snuff and chewing tobacco users on average get systemic nicotine doses that per unit is 3.6 mg and 4.6 mg respectively. Important to note is however, that only a part of the nicotine present in a tobacco dose is delivered and absorbed systemically. The concentration of nicotine in tobacco may be in the range of 1 - 3 % and the tobacco content in a portion of chewing tobacco may be 1 g. The corresponding figures for wet snuff are 0.5 - 1 % and 0.5 - 1 g respectively. Thus a portion of snuff or chewing tobacco can contain between 2.5 mg and 30 mg of nicotine. A sachet dose of the cellulose based carrier system described here may therefore also contain nicotine in the range 2.5 to 30 mg. The delivered dose of nicotine should preferably be 1 to 8 mg of nicotine. This dose may be released during a time span of 0 - 40 minutes after application of the sachet, more preferably during the time interval 0 - 20 minutes.

The advantage of the above delivery system is that nicotine is delivered in a white or slightly grey sachet, not as heavy brown snuff. That will provide for a nicotine delivery without the yellowing of teeth, the formation of brown saliva and the non-pleasant removal of the used brown snuff from the mouth.

Optional addition of buffering agents

Buffering agents may be optionally added mainly, but not exclusively, in formulations of the present invention intended for buccal delivery.

Absorption of nicotine from the oral cavity to the systemic circulation is dependent on the pH of the saliva and the pKa of nicotine, which is about 7.8. Assuming a pH of the saliva of 6.8 only about 10% of the nicotine in saliva will be in the free base form. Thus, in order to promote absorption of nicotine in a free base form, which is the form predominantly absorbed through the mucosa, the pH of the saliva must be increased. At a pH of 8.8 8 about 90% of the nicotine in saliva will then be in the free base form.

Hence and according to the invention, the present nicotine-containing pharmaceutical composition may be alkalised by buffering and/or pH regulation. This may be achieved by including physiologically acceptable buffering substances or agents, or by other means. With other means it is intended to include buffering by any component in the product, which may not normally act as a buffering agent, such as a self-buffering additive and/or pH regulating forms of nicotine.

By buffering and/or pH regulation thereby increasing the pH of the saliva the uptake of nicotine is changed, e g increased compared to the nicotine uptake when the saliva is not alkalised by buffering and/or pH regulation. Also, since the transmucosal uptake of nicotine in the oral cavity according to the invention is faster than for nicotine not being buffered and/or pH regulated according to the invention, less nicotine will be swallowed and reach the gastrointestinal (GI) tract. The nicotine that reaches the GI tract will be subjected to first pass metabolism, which reduces the total amount of intact nicotine absorbed. This means that the bioavailability of nicotine that is not co-administered with a buffer according to the invention will generally be lower than when administered together with a buffer.

For buffering may be used one or more buffering agents selected from the group consisting of carbonates including bicarbonate or sesquicarbonate, glycinate, phosphate, glycerophosphate or citrate of an alkali metal, such as potassium or sodium, or ammonium; sodium hydroxide, potassium hydroxide, calcium oxide, and mixtures thereof.

Further embodiments may use trisodium or tripotassium citrate, and mixtures thereof.

Still further embodiments may comprise different phosphate systems, such as trisodium phosphate, disodium hydrogen phosphate; and tripotassium phosphate, dipotassium hydrogen phosphate, and calcium hydroxide, sodium glycinate; and mixtures thereof.

Alkali metal carbonates, glycinates and phosphates are preferred buffering agents.

The pH regulation may also be obtained by using pH-regulating forms of nicotine, e g nicotine free base.

The amount of the buffering agent or agents in the liquid pharmaceutical formulation is preferably sufficient in the specific embodiments to raise the pH of the saliva to above 7, as specified above and, to maintain the pH of the saliva in the oral cavity above 7, e g pH 7 - 11. Otherwise expressed the liquid pharmaceutical formulation should be alkalised by buffering and/or pH regulation in such a way that upon administration to a subject the pH of the liquid of the oral cavity of the subject is transiently increased by about 0.3 - 4 pH units, preferably by about 0.5 - 2.5 pH units. The amount of buffering agent(s) required to achieve such an increase in pH is readily calculated by a person skilled in the art.

Other additives to the nicotine-containing composition

Other additives may be added optionally to the present nicotine-containing composition.

Optional additives comprise one or more stabilizing additives, such as those selected from the group consisting of antioxidants including vitamin E, i e tocopheroles, vitamin C, i e ascorbic acid and its salts, sodium pyrosulfite, butylhydroxytoluene, butylated hydroxyanisole; and preservatives including parabenes, benzalkonium chloride, chlorbutanol, benzyl alcohol, beta-phenylethyl alcohol, cetylpyridinium chloride, citric acid, tartaric acid, lactic acid, malic acid, acetic acid, benzoic acid, and sorbic acid and their salts; and chelating agents, such as EDTA; and galates, such as propyl galate.

Further optional additives comprise one or more additives selected from the group consisting of:

- enhancers, such as azone;
- vitamins, such as vitamins B, C and E;
- minerals, such as fluorides, especially sodium fluoride, sodium monofluoro phosphate and stannous fluoride;
- anti-odours, such as zinc and cyclodextrins;
- propellants, such as 1,1,2,2-tetrafluoroethane (HFC-134a), optionally being liquefied, and 1,1,1,2,3,3,3-heptafluororpropane (HFC-227), optionally being liquefied;
- sweeteners including one or more synthetic sweetening agents and/or natural sugars, such as those selected from the groups consisting of e g saccharin and its sodium and calcium salts, aspartame, acesulfame and its potassium salt, thaumatin, glycyrrhizin, sucralose, dihydrochalcone, alitame, miraculin, monellin and stevside.
- polyhydric alcohols such as sorbitol, xylitol, mannitol and glycerol;
- monosaccharides including glucose (also called dextrose), fructose (also called laevulose) and galactose;
- disaccharides including saccharose (also called sucrose), lactose (also called milk sugar) and maltose (also called malt sugar);
- mixtures of sugars including liquid glucose syrup e g starch hydrolysates containing a mixture of chiefly dextrose, maltose, dextrins and water, invert sugar syrup e g sucrose inverted by invertase containing a mixture of dextrose, laevulose and water, high sugar content syrups such as treacle, honey and malt extract;

and mixtures thereof;

- flavoring and/or aromatizing agents, such as those selected from the group consisting of essential oils obtained by distillations, solvent extractions or cold expressions of fresh or dried flowers, buds, leaves, stems, fruit, seeds, peel, bark, or root e g oil of peppermint, spearmint, eucalyptus, wintergreen, niaouli, clove, cardamom, cinnamon, bitter almond, coriander, caraway, ginger, juniper, orange, bitter orange, lemon, grapefruit, mandarine, bergamot, thyme, fennel and rosemary;
- natural flavors and aroma agents including either diluted solutions of essential oils or concentrates of flavor components with natural origin from e g fruits, berries, nuts, spices, mints, tobacco, cocoa, coffee, tea, vanilla, liquorice, caramel, toffee, honey, wine, liquors and brews;
- synthetic flavors and aroma agents consisting of mixtures of chemicals comprising hydrocarbons, alcohols, aldehydes, esters, ketones, ethers and oxides blended to match the natural flavor of e g fruits, berries, nuts, spices, mints, tobacco, cocoa, coffee, tea, vanilla, liquorice, caramel, toffee, honey, wine, liquors or brews;
- and mixtures thereof.

## **Examples**

### Example 1. Purifying cellulose

This example describes a method to extract cellulose from algal, bacterial or fungal sources without limiting the invention thereto. 500 g of dry Cladophora species algae was bleached with 180 g of $NaClO_2$ in 0.51 acetic acid. The mixture was diluted to 5 1, poured into a plastic bag and stored in a water bath at 60° C for 3 hours. The algae were washed until neutrality (pH~7) as indicated by coloration of a pH paper and filtered. NaOH 0.5 M, 3 1, was added to the remainder and the resultant product was stored at 60° C in a water-bath overnight. The resultant pulp was washed till neutrality, filtered and dried. Dry, purified algae were ground in a mill prior to acid hydrolysis. To 50 g of dry, purified algae 11 of 5% HCl was added, and the suspension was heated to boiling. Once boiling, it was removed from the heat, and the slurry was allowed to stand overnight. Then the cellulose was washed to neutrality again and spray-dried.

Primary characteristics of some cellulose powders are shown in below Table 3.

**Table 3**. Primary characteristics of different cellulose powders

| Cellulose powder from | Crystallinity index, % | BET[a] surface area $N_2$, $m^2$/g | Pore volume[b] $cm^3$/g |
|---|---|---|---|
| Seed organisms[c] | 82.2 | 0.96 | 0.0030[f] |
| Brown Algae[d] | 81.7 | 5.76 | 0.0250[g] |

(continued)

| Cellulose powder from | Crystallinity index, % | BET[a] surface area N$_2$, m$^2$/g | Pore volume[b] cm$^3$/g |
|---|---|---|---|
| Cladophora[e] | 95.2 | 94.7 | 0.5540[h] |

[a] Surface area according to the Brunauer, Emmet and Teller method.

[b] Pore volume refers to the total volume of pores in the cellulose particles filled with nitrogen at relative partial pressures of 0.9862[f], 0.9858[g] and 0.9799[h] respectively.

[c] Commercially available microcrystalline cellulose (Avicel PH 102®)

[d] Algiflor, Danisco, France. The Algiflor was a blend of five brown algae species: Laminaria digitata, Lessonia nigrescens, Macrocystis pyrifera, Ascophyllum nodosum, and Fucus serratus.

[e] Cladophora glomerata was harvested in the Baltic Sea and was pressed to a dryness of about 25 % and dried in room temperature.

Of interest for the present invention is primarily cellulose particles having a pore volume above 0,01 cm$^3$/g.

Example 2. Manufacture of nicotine-cellulose matrix

This example describes without limiting the invention to two ways of adding nicotine to algal cellulose.

Example 2A. By rotary evaporation

Cellulose powder was washed with ethanol and added to a rotary evaporator. A nicotine solution in ethanol was added and mixed thoroughly with the cellulose powder. After that, the ethanol was evaporated at 200 mbar and 60° C. Maximum deposition of nicotine with the cellulose was approximately 50% of the total weight of the nicotine-cellulose matrix.

Example 2B. In contact with air saturated with nicotine

Cellulose powder in shallow open vessels was exposed to nicotine vapour at 60° C for five days. Maximum deposition of nicotine with the cellulose was approximately 20% of the total weight of the nicotine-cellulose matrix.

The above two ways of adding nicotine to algal cellulose is useful also for adding nicotine to cellulose of other origin.

Example 3. Manufacture of inhaler

Example 3A.

Nicotine-cellulose matrix was compressed into a suitable disc or tablet providing for a flow resistance of 0.2-0.6 kPa at an air flow rate of 1000 ml/min. The porous disc/tablet was placed in an inhaling device. When a user puffed on the inhaling device gaseous nicotine was released to the air stream sucked through it.

Alternatively, nicotine-cellulose matrix was mixed with powdered cellulose and compressed into a suitable disc or tablet providing for a flow resistance of 0.2-0.6 kPa at a flow rate of 1000 ml/min. The porous disc/tablet was placed in an inhaling device. When the user puffed on the inhaling device nicotine was released to the air sucked through it. The composition of the disc or tablet was per below Table 4.

**Table 4.** Composition of the disc or tablet

| **Active ingredient** | |
|---|---|
| Nicotine-cellulose matrix 40% | 75 mg |
| **Other ingredients** | |
| Powdered cellulose | 100 mg |
| Levomentol as flavorant | 3 mg |

Example 3B.

This example was carried out as Example 3A with the sole exception that the disc/tablet was substituted for nicotine-cellulose matrix placed between two permeable discs placed in a small cylinder.

Example 4. Manufacture of transdermal patch

Example 4A.

4.5 g of nicotine-cellulose matrix was suspended in 40.0 g demineralised water. This suspension was added together with 6.0 g propylene glycol to 26.0 g Polyvidon 90 gel as per below Table 5 to obtain a drug gel. The drug gel was solvent cast onto a polyester sheet of 75 $\mu$m thickness by means of a coating machine in a layer of 1.0 mm. The gel layer was dried at room temperature. Adhesive preparation I as per below Table 6 was solvent cast onto a siliconized polyethylene foil and after drying laminated onto the gel-coated sheet by means of a steel cylinder.

The drug-adhesive layer was covered by a release liner and the sheets were kept in heat-sealed pouches until use.

The resulting sheet with backing layer, drug-adhesive layer and release liner was 0.5 mm thick and the concentration of nicotine was about 1.4 mg/cm$^2$.

| Table 5. Composition and manufacturing of Polyvidon 90 gel | | |
|---|---|---|
| Composition: | Ethanol 99.9% | 226 g |
| | Demineralised water | 200 g |
| | Polyvidon | 125 g |
| Manufacturing: | Ethanol and water was mixed and Polyvidon 90 was added gradually under stirring. The stirring continued for 10 further minutes. Then the gel was left to swell for at least 16 hours. | |

| Table 6. Composition of Adhesive preparation I | |
|---|---|
| Polyisobutylene Oppanol 10 | 1.0 g |
| Polyisobutylene Oppanol 50 | 1.5 g |
| Polyisobutylene Oppanol 120 | 2.0 g |
| Hexane | 30.0 ml |
| Liquid paraffin | 5.5 g |

Example 4B.

6.0 g of nicotine-cellulose matrix was added to 68.4 g of Adhesive preparation II according to below Table 7. Then 2.4 g nicotine was added to provide a drug gel. The drug gel was solvent cast onto a polyester sheet of 75 $\mu$m thickness by means of a coating machine in a layer of 0.8 mm and the gel layer was dried at room temperature. Adhesive preparation I as per above Table 6 was laminated onto the gel coated sheet. The lamination was accomplished by solvent casting Adhesive preparation I onto a siliconized polyethylene foil in a 100 $\mu$m layer, drying at room temperature and pressing of the layer onto the dry drug gel layer by means of a steel cylinder after which the polyethylene foil was removed.

The drug-adhesive layer was covered by a release liner and the sheets were kept in heat-sealed pouches until use.

The resulting sheet with backing layer, drug-adhesive layer and release liner was 0.29 mm thick and the concentration of nicotine was about 2.1 mg/cm$^2$.

| Table 7. Composition of Adhesive preparation II | |
|---|---|
| Polyisobutylene Oppanol 10 | 2.0 g |
| Polyisobutylene Oppanol 50 | 1.5 g |
| Polyisobutylene Oppanol 120 | 1.0 g |
| Hexane | 30.0 ml |
| Liquid paraffin | 2.0 g |

Example 5. Manufacture of sublingual tablets

This example describes without limiting the invention as applied to sublingual tablets. The method of manufacture may be any suitable method known in the art. The tablets have a preferred composition as per below Table 8.

For direct compression the powders are mixed and compressed in 6 mm punches with a weight of 90 mg.

**Table 8.** Composition of sublingual tablets

| Active ingredients | 4 mg unit formula (mg) | 2 mg unit formula (mg) |
|---|---|---|
| Nicotine-cellulose matrix 40% | 10 | 5 |
| **Other ingredients** | | |
| Mannitol | 51 | 51 |
| Microcrystalline cellulose | 5 | 15 |
| Povidone | 14 | 9 |
| Methylcellulose | 4 | 4 |
| Silicified microcrystalline cellulose | 2.5 | 2.5 |
| Lemon flavour | 1.5 | 1.5 |
| Special compound | 0.5 | 0.5 |
| Magnesium stearate | 0.9 | 0.9 |
| Aspartame | 0.7 | 0.7 |

Example 6. Manufacture of chewing gums

This example describes without limiting the invention to the manufacture of different uncoated chewing gums, so called cores, according to the invention. The cores can be coated and nicotine can be included in the coating.

Objective

The objective of this example is to provide a core suitable for a chewing gum product according to the invention. The nicotine is incorporated as free base absorbed into and/or adsorbed on Cladophora species cellulose.

Principle

The core is formed by a mixing, rolling and scoring process or by a compression process.

Composition of the cores

The core composition is preferably according to below Tables 9 and 10.

**Table 9.** Composition of cores manufactured by a tablet compression process.

| Active ingredients | 4 mg unit formula (mg) | 2 mg unit formula (mg) |
|---|---|---|
| Nicotine-cellulose matrix 40% | 10 | 5 |
| **Other ingredients** | | |
| Chewing gum base for compression[a] | 500 | 500 |
| Xylitol | 211 | 221 |
| Sorbitol | 100 | 100 |
| Encapsulated peppermint oil | 100 | 100 |
| Sodium carbonate anhydr. | 30 | 20 |
| Sodium bicarbonate | - | 10 |
| Magnesium stearate | 15 | 15 |
| Talcum | 15 | 15 |
| Magnesium oxide | 5 | 5 |
| Acesulfame K | 2 | 2 |
| Aspartame | 2 | 2 |
| [a] Cafosa Gum S/A | | |

**Table 10.** Composition of cores manufactured by mixing rolling and scoring

| Active ingredients | 4 mg Unit formula (mg) | 2 mg Unit formula (mg) |
|---|---|---|
| Nicotine-cellulose matrix 40 % | 10 | 5 |
| **Other ingredients** | | |
| Chewing gum base[a] | 620 | 620 |
| Xylitol | 310 | 312 |
| Peppermint oil | 30 | 30 |
| Sodium carbonate anhydr. | 25 | 20 |
| Sodium bicarbonate | 5 | 10 |
| Acesulfame K | 2 | 2 |
| Levomenthol | 2 | 2 |
| Magnesium oxide | 2 | 2 |
| [a] Cafosa Gum S/A | | |

Procedures for core manufacturing

I) Mixing, rolling and scoring

Mixing, rolling and scoring are performed by a conventional procedure. Double sigma blade mixers are used for mixing the gum base with the other components of the formulation. The gum base is softened in a mixer. Through heat from a heating jacket and mixing, the gum base becomes plastic. The so softened base is mixed with the liquid components, e g flavours, liquid, sorbitol and glycerol, and the solid materials, e g nicotine in any solid form, buffer, bulk sweetener, colour as a powder mixture. The warm mass is discharged from the mixer in form of loaves stacked on trays on a truck and stored in a conditioned area until the next step starts. This procedure is intended for cooling the gum.

After this, the rolling and scoring takes place. The gum is extruded into a thick sheet, which is rolled by multiple sets of calender rolls to the correct thickness. The scoring rolls, usually two sets, cut the sheet into correct size.

The sheets are then transferred to a conditioned area on trays, where the sheets are cooled to make them brittle enough to be broken. The conditioned gum sheets are then passed through a breaker, which is a rotating drum that parts the sheets into separate pieces of gum along the scores.

At a sorting stage deformed gums are sorted away. The accepted gums are passed through a metal detector.

II) Compression

Chewing gums produced by compression, usually being a dry method, i e tabletted gums, are made out of a special gum base. High velocity mixers may be used for granulation to give correctly sized particles of the mixture. This mixture is then compressed in a tablet machine.

At a sorting stage deformed gums are sorted away. The accepted gums are passed through a metal detector.

Example 7. Manufacture of buccal sachet

A sachet of an appropriate woven or non-woven semi-permeable cloth material, with an approximate dimension of 15 x 20 mm, is filled with a predetermined amount of nicotine-cellulose matrix. See below Table 11. In order to obtain a formulation with a suitable mass, an inert filling material may be added to the sachet. Such a filling material acts as bulking agent to increase the physical size of the dosage form. The filling material can be powdered cellulose with a suitable particle size. The sachet is sealed by gluing or by heat sealing.

**Table 11.** Composition of a buccal sachet

| | Unit formula (mg) |
|---|---|
| **Active ingredient** | |
| Nicotine-cellulose mixture 40% | 15 |

(continued)

| Other ingredients | |
|---|---|
| Powdered cellulose | 300 |
| Levomenthol as flavorant | 2 |

Example 8. Stability test of nicotine-cellulose matrices prepared from nicotine and particles of Cladophora species cellulose and microcrystalline cellulose

| | |
|---|---|
| Storage condition: | 25 °C and 60 % relative humidity. |
| Package: | Glass jars with Teflon lined screw caps. |
| Analytical method: | Liquid chromatographic determination of nicotine and related compounds (i e identified impurities and degradation products). |
| Sampling times: | At start of the study (time 0) and after storage for 1 month and for 3 months. |

| Sample | Time, months | Content of nicotine, %(w/w) | Recovery of initial nicotine, % | Content of sum of related substances, % |
|---|---|---|---|---|
| Nicotine/Cladophora cellulose matrix | 0 | 53.8 | 100.0 | b.q. |
| | 1 | 55.2 | 102.6 | 1.34 |
| | 3 | 54.7 | 101.7 | 2.88 |
| Nicotine/ Microcrystalline cellulose matrix | 0 | 18.25 | b.q. | b.q. |
| | 1 | 16.63 | 91.1 | 5.4 |
| | 3 | 10.99 | 60.2 | 23.6 |
| b.q. means content below limit of quantitation | | | | |

The present nicotine-cellulose matrix compositions are useful in therapy, such as in therapy for obtaining reduction of the urge to smoke or otherwise use tobacco containing material and/or for providing a sense of smoking satisfaction without smoking, comprising administering to a subject the present nicotine-containing pharmaceutical composition.

Said therapy may also be combined with one or more other methods for obtaining reduction of the urge to smoke or otherwise use tobacco containing material and/or for providing a sense of smoking satisfaction without smoking. Said one or more other methods may be selected from the group consisting of administration through chewing gums, nasal sprays, transdermal patches, lozenges, tablets and parenteral means or methods, subcutaneous means or methods, intravenous means or methods, rectal means or methods, vaginal means or methods and transmucousal means or methods; or use of tobacco. Said one or more other methods may comprise administration of nicotine.

The present nicotine-cellulose matrix containing compositions are also useful in treating a disease selected from the group consisting of addiction to tobacco or nicotine, Alzheimer's disease, Crohn's disease, Parkinson's disease, Tourette's syndrome, ulcerative colitis; and weight control.

## Claims

1. A nicotine-containing pharmaceutical composition, **characterized in that** it comprises nicotine being reversibly absorbed into and/or adsorbed onto cellulose from algae, bacteria and/or fungi.

2. A nicotine-containing pharmaceutical composition according to claim 1, **characterized in that** at least part of the cellulose is obtained from one or more algae selected from
blue-green algae (Cyanophyta), such as Anabaena and Nostoc punctiformae;

green algae (Chlorophyta), such as Chladophora glomerata and rupestris, Chaetomorpha melagonium, Enteromorpha, Ulva lactuca, Oocystis, e g solitaria and apiculata, and Valonia, e g ventricosa, and Chara corallina;
gold algae (Chrysophyta), such as Vaucheria;
dinoflagellates (Pyrrophyta), such as Crypthecodinium cohnii, Gonyaulax polyedra, Scrippsiella hexapraecingula, Dinobryon and Peridinium;
brown algae (Phaeophyta), such as Lessonia nigrescens, Macrocystis pyrifera, Ascophyllum nodosum, Fucus serratus, Halidrys siliquosa, Himanthalia lorea, Pelvetia canaliculata, Laminaria digitata, and
red algae (Rhodophyta), such as Griffithsia flusculosa, Porphyra, Ptilota plumosa, Rhodymenia palmate and Erythrocladia subintegra.

3. A nicotine-containing pharmaceutical composition according to claim 1, **characterized in that** at least part of the cellulose is obtained from bacteria selected from Acetobacter, such as xylinum, pasteurianus, aceti and acetigenus;
Achromobacter;
Aerobacter;
Agrobacterium, such Agrobacterium tumefaciens;
Alcaligenes;
Polyspondium pallidum;
Pseudomonas;
Rhizobium, such as Rhizobium leguminosarum;
Sarcina;
Sphaerofilus natans;
Xanthomonas campestris, and
Zoogloega.

4. A nicotine-containing pharmaceutical composition according to claim 1, **characterized in that** at least part of the cellulose is obtained from fungi selected from Achlya bisexualis;
Saprolegnia, such as parasitica and monoica;
Dictyostelium, such as Dictyostelium discoideum;
Phytophtora, such as parasitica var. nicotianae and cactorum;
Microdochium nivale;
Ophiostoma ulmi;
Colletotrichum lindemuthianum, and
Pythium, such as aphanidermatum, butleri and ultimatum.

5. A nicotine-containing pharmaceutical composition according to any of claims 1-4, **characterized in that** the nicotine is selected from nicotine salts, preferably tartrate, hydrogen tartrate, hydrochloride, acetate or salicylate, the free base form of nicotine, nicotine in any non-covalent binding, and mixtures thereof.

6. A nicotine-containing pharmaceutical composition according to any of claims 1-5, **characterized in that** it further comprises one or more flavoring and/or aromatizing agents, such as those selected from the group consisting of essential oils obtained by distillations, solvent extractions or cold expressions of fresh or dried flowers, buds, leaves, stems, fruit, seeds, peel, bark, or root e g oil of peppermint, spearmint, eucalyptus, wintergreen, niaouli, clove, cardamom, cinnamon, bitter almond, coriander, caraway, ginger, juniper, orange, bitter orange, lemon, grapefruit, mandarine, bergamot, thyme, fennel and rosemary; natural flavors and aroma agents including either diluted solutions of e g essential oils or concentrates of flavor components with natural origin from e g fruits, berries, nuts, spices, mints, tobacco, cocoa, coffee, tea, vanilla, liquorice, caramel, toffee, honey, wine, liquors and brews, and mixtures thereof.

7. A nicotine-containing pharmaceutical composition according to any of claims 1-6, **characterized in that** it further comprises one or more stabilizing additives, such as those selected from the group consisting of antioxidants including vitamin E, i e tocopheroles, vitamin C, i e ascorbic acid and its salts, sodium pyrosulfite, butylhydroxytoluene, butylated hydroxyanisole; and preservatives including parabenes, benzalkonium chloride, chlorbutanol, benzyl alcohol, beta-phenylethyl alcohol, cetylpyridinium chloride, citric acid, tartaric acid, lactic acid, malic acid, acetic acid, benzoic acid, and sorbic acid and their salts; and chelating agents, such as EDTA; and galates, such as propyl galate.

8. A nicotine-containing pharmaceutical composition according to any of claims 1 - 7, **characterized in that** it further comprises one or more additives selected from the group consisting of:

thickening agents, such as natural, semisynthetic or synthetic polymers, e g starch and starch derivatives, cellulose and cellulose derivatives, polyethylene glycols and derivatives thereof, polyacrylates, and polyvinyl esters and ethers;

enhancers, such as azone;

vitamins, such as vitamins B, C and E;

minerals, such as fluorides, especially sodium fluoride, sodium monofluoro phosphate and stannous fluoride;

anti-odours, such as zinc and cyclodextrins;

propellants, such as 1,1,2,2-tetrafluoroethane (HFC-134a), optionally being liquefied, and 1,1,1,2,3,3,3-heptafluororpropane (HFC-227), optionally being liquefied; sweeteners including one or more synthetic sweetening agents and/or natural sugars, such as those selected from the groups consisting of artificial sweeteners e g saccharin and its sodium and calcium salts;

aspartame, acesulfame and its potassium salt, thaumatin, glycyrrhizin, sucralose, dihydrochalcone, alitame, miraculin, monellin and stevside;

polyhydric alcohols such as sorbitol, xylitol, mannitol and glycerol;

monosaccharides including glucose also called dextrose, fructose also called laevulose and galactose;

disaccharides including saccharose also called sucrose, lactose also called milk sugar and maltose also called malt sugar;

mixtures of sugars including liquid glucose syrup e g starch hydrolysates containing a mixture of chiefly dextrose, maltose, dextrins and water, invert sugar syrup e g sucrose inverted by invertase containing a mixture of dextrose, laevulose and water, high sugar content syrups such as treacle, honey and malt extract;

and mixtures thereof.

9. A nicotine-containing pharmaceutical composition according to any of claims 1 - 8, **characterized in that** it is alkalised by buffering and/or pH regulation by the use of one or more buffering agents selected from the group consisting of a carbonate, such as mono-carbonate, bicarbonate or sesquicarbonate; glycinate, phosphate, glycerolphosphate, acetate, gluconate or citrate of an alkali metal, such as potassium or sodium, or of ammonium, and mixtures thereof; and/or by the use of pH regulating agents, such as agents selected from the group consisting of sodium hydroxide, potassium hydroxide, calcium hydroxide and calcium oxide; and/or by using at least partly pH regulating forms of nicotine.

10. A nicotine-containing pharmaceutical composition according to any of claims 1 - 9, **characterized in that** it is comprised in an inhaling device, a transdermal patch, a nasal spray, a mouth spray, an inhaling device or an oral dosage form, such as a buccal sachet, a lozenge, such as a sublingual lozenge, a tablet or a powder.

11. A method for prepaying a nicotine-containing pharmaceutical composition according to any of claims 1 - 10, **characterized in that** the cellulose is exposed to nicotine vapour or to an alcoholic solution of nicotine or nicotine salt.

12. A method for accomplishing reduction of the use of tobacco-containing material and/or for providing a sense of smoking satisfaction without smoking, or for attaining weight control comprising administering to a subject a nicotine-containing pharmaceutical composition according to any of claims 1 - 10.

13. A method for accomplishing reduction of the use of tobacco-contatning material and/or for providing a sense of smoking satisfaction without smoking or for attaining weight control, wherein a method according to claim 12 is used in combination with one or more other methods for accomplishing redUction of the use of tobacco-containing material and/or for providing a sense of smoking satisfaction without smoking or for attaining weight control.

14. A nicotine-containing pharmaceutical composition according to any of claims 1 - 10 for use in a therapy, being a treatment of a disease selected from the group consisting of addiction to tobacco or to nicotine, Alzheimer's disease, Crohn's disease, Parkinson's disease, Tourette's syndrome, ulcerative colitis; and weight control.

15. Use of nicotine and cellulose from algAe, bacteria and/or fungi for the manufacturing of a nicotine-containing pharmaceutical composition according to any of claims 1 - 10 for the treatment of a disease selected from the group consisting of addiction to tobacco or to nicotine, Alzheimer's disease, Crohn's disease, Parkinson's disease, Tourette's syndrome, ulcerative colitis; and weight control.

**Patentansprüche**

1. Nikotin enthaltende pharmazeutische Zusammensetzung, **dadurch gekennzeichnet, dass** sie Nikotin umfasst, das reversibel in Cellulose von Algen, Bakterien und/oder Pilzen absorbiert wird und/oder darauf adsorbiert wird.

2. Nikotin enthaltende pharmazeutische Zusammensetzung nach Anspruch 1, **dadurch gekennzeichnet, dass** mindestens ein Teil der Cellulose von einer oder mehreren Algen erhalten wird, die ausgewählt sind aus
Blaugrünalgen (Cyanophyta), wie beispielsweise Anabaena und Nostoc punctiformae;
Grünalgen (Chlorophyta), wie beispielsweise Chladophora glomerata und rupestris, Chaetomorpha melagonium, Enteromorpha, Ulva lactuca, Oocystis, zum Beispiel solitaria und apiculata, und Valonia, zum Beispiel ventricosa, und Chara corallina;
Goldalgen (Chrysophyta), wie beispielsweise Vaucheria;
Dinoflagellaten (Pyrrophyta), wie beispielsweise Crypthecodinium cohnii, Gonyaulax polyedra, Scrippsiella hexapraecingula, Dinobryon und Peridinium;
Braunalgen (Phaeophyta), wie beispielsweise Lessonia nigrescens, Macrocystis pyrifera, Ascophyllum nodosum, Fucus serratus, Halidrys siliquosa, Himanthalia lorea, Pelvetia canaliculata, Laminaria digitata und
Rotalgen (Rhodophyta), wie beispielsweise Griffithsia flusculosa, Porphyra, Ptilota plumosa, Rhodymenia palmate und Erythrocladia subintegra.

3. Nikotin enthaltende pharmazeutische Zusammensetzung nach Anspruch 1, **dadurch gekennzeichnet, dass** mindestens ein Teil der Cellulose von Bakterien erhalten wird, die ausgeählt sind aus Acetobacter, wie beispielsweise xylinum, pasteurianus, aceti und acetigenus;
Achromobacter;
Aerobacter;
Agrobakterium, wie Agrobakterium tumefaciens;
Alcaligenes;
Polyspondium pallidum;
Pseduomonas;
Rhizobium, wie beispielsweise Rhizobium leguminosarum;
Sarcina;
Sphaerofilus natans;
Xanthomonas campestris und
Zoogloega.

4. Nikotin enthaltende pharmazeutische Zusammensetzung nach Anspruch 1, **dadurch gekennzeichnet, dass** mindestens ein Teil der Cellulose von Pilzen erhalten wird, die ausgewählt sind aus
Achlya bisexualis;
Saprolegnia, wie beispielsweise parasitica und monoica;
Dictyostelium, wie beispielsweise Dictyostelium discoideum;
Phytophtora, wie beispielsweise parasitica var. nicotianae und cactorum;
Microdochium nivale;
Ophiostoma ulmi;
Colletotrichum lindemuthianum und
Pythium, wie beispielsweise aphanidermatum, butleri und ultimatum.

5. Nikotin enthaltende pharmazeutische Zusammensetzung nach einem der Ansprüche 1 bis 4, **dadurch gekennzeichnet, dass** das Nikotin ausgewählt ist aus Nikotinsalzen, vorzugsweise Tartrat, Wasserstofftartrat, Hydrochlorid, Acetat oder Salicylat, der freien Basenform von Nikotin, Nikotin in einer nicht kovalenten Bindung und Gemischen davon.

6. Nikotin enthaltende pharmazeutische Zusammensetzung nach einem der Ansprüche 1 bis 5, **dadurch gekennzeichnet, dass** sie weiter umfasst: eine oder mehrere Geschmacks- und/oder Aromastoffe, wie beispielsweise die aus der Gruppe bestehend aus etherischen Ölen ausgewählten, die durch Destillation, Lösungsmittelextraktion oder Kaltexpression von frischen oder getrockneten Blumen, Knospen, Blättern, Stengeln, Früchten, Samen, Schale, Rinde oder Wurzel erhalten werden, zum Beispiel Öl aus Pfefferminze, grüne Minze, Eukalyptus, Immergrün, Niaouli, Nelke, Kardamom, Zimt, Bittermandel, Koriander, Kümmel, Ingwer, Wacholder, Orange, Bitterorange, Zitrone, Grapefruit, Manadarine, Bergamotte, Thymian, Fenchel und Rosemarin; natürliche Geschmacksstoffe und Aromastoffe, einschließlich entweder verdünnte Lösungen von zum Beispiel etherischen Ölen oder Konzentraten von Geschmackskomponenten mit natürlichem Ursprung zum Beispiel von Früchten, Beeren, Nüssen, Gewürzen, Minzen, Tabak, Kakao, Kaffee, Tee, Vanille, Süßholz, Karamel, Karamell, Honig, Wein, alkoholischen Getränken und Gebräuen, und Gemische davon.

7. Nikotin enthaltende pharmazeutische Zusammensetzung nach einem der Ansprüche 1 bis 6, **dadurch gekennzeichnet, dass** sie weiter umfasst: ein oder mehrere stabilisierende Additive, wie beispielsweise die aus der Gruppe bestehend aus Antioxidationsmitteln ausgewählten, einschließlich Vitamin E, d.h. Tocopherole, Vitamin C, d.h. Ascorbinsäure und deren Salze, Natriumpyrosulfit, Butylhydroxytoluol, butyliertes Hydroxyanisol; und Konservierungsstoffe, einschließlich Parabene, Benzalkoniumchlorid, Clorbutanol, Benzylalkohol, beta-Phenylethylalkohol, Cetylpyridiniumchlorid, Zitronensäure, Weinsäure, Milchsäure, Apfelsäure, Essigsäure, Benzoesäure und Sorbinsäure und deren Salze; und Chelatbildner, wie beispielsweise EDTA; und Galate, wie beispielsweise Propylgalat.

8. Nikotin enthaltende pharmazeutische Zusammensetzung nach einem der Ansprüche 1 bis 7, **dadurch gekennzeichnet, dass** sie weiter eine oder mehrere Additive umfasst, die ausgewählt sind aus der Gruppe bestehend aus:

Verdickungsmitteln, wie beispielsweise natürlichen, halbsynthetischen oder synthetischen Polymeren, zum Beispiel Stärke und Stärkederivate, Cellulose und Cellulosederivate, Polyethylenglycole und Derivate davon, Polyacrylate und Polyvinylester und -ether;
Verstärker, wie beispielsweise Azon;
Vitaminen, wie beispielsweise Vitamin B, C und E;
Mineralstoffen, wie beispielsweise Fluoriden, insbesondere Natriumfluorid, Natriummonofluorphosphat und Zinnfluorid;
geruchshemmenden Stoffen, wie beispielsweise Zink und Cyclodextrinen;
Treibmitteln, wie beispielsweise 1,1,2,2-Tetrafluorethan (HFC-134a), wahlweise verflüssigt, und 1,1,1,2,3,3,3-Heptafluorpropan (HFC-227), wahlweise verflüssigt;
Süßstoffen, einschließlich einem oder mehreren synthetischen Süßstoffen und/oder natürlichen Zuckern, wie beispielsweise den aus den Gruppen bestehend aus künstlichen Süßstoffen, z.B. Saccharin und dessen Natrium- und Calciumsalzen, ausgewählten;
Aspartam, Acesulfam und dem Kaliumsalz davon, Thaumatin, Glycyrrhizin, Sucralose, Dihydrochalcon, Alitam, Miraculin, Monellin und Stevsid;
mehrwertigen Alkoholen, wie beispielsweise Sorbit, Xylit, Mannit und Glycerin;
Monosacchariden, einschließlich Glucose, auch als Dextrose bezeichnet, Fruktose, auch als Laevulose bezeichnet, und Galactose;
Disacchariden, einschließlich Saccharose, auch als Sucrose bezeichnet, Lactose, auch als Milchzucker bezeichnet, und Maltose, auch als Malzzucker bezeichnet;
Gemischen aus Zuckern, einschließlich flüssigem Glucosesirup, z.B. Stärkehydrolysaten, enthaltend ein Gemisch aus hauptsächlich Dextrose, Maltose, Dextrinen und Wasser, Invertzuckersirup, z.B. Sucrose, umgekehrt durch Invertase, enthaltend ein Gemisch aus Dextrose, Laevulose und Wasser, Sirupen mit hohem Zuckergehalt, wie beispielsweise Melasse, Honig und Malzextrakt;

und Gemischen davon.

9. Nikotin enthaltende pharmazeutische Zusammensetzung nach einem der Ansprüche 1 bis 8, **dadurch gekennzeichnet, dass** sie alkalisiert werden durch Pufferung und/oder pH-Wert-Regulation unter Verwendung von einem oder mehreren Puffermitteln, die ausgewählt sind aus der Gruppe bestehend aus einem Carbonat, wie beispielsweise Monocarbonat, Bicarbonat oder Sesquicarbonat; Glycinat, Phosphat, Glycerolphosphat, Acetat, Gluconat oder Citrat eines Alkalimetalls, wie beispielsweise Kalium oder Natrium, oder eines Ammoniums, und Gemischen davon; und/oder unter Verwendung von pH-Wert-Regulationsmitteln, wie beispielsweise Mitteln, ausgewählt aus der Gruppe bestehend aus Natriumhydroxid, Kaliumhydroxid, Calciumhydroxid und Calciumoxid; und/oder unter Verwendung von mindestens teilweise pH-reguliuerenden Formen von Nikotin.

10. Nikotin enthaltende pharmazeutische Zusammensetzung nach einem der Ansprüche 1 bis 9, **dadurch gekennzeichnet, dass** sie in einer Inhalierungseinrichtung, einem transdermalen Pflaster, einem Nasenspray, einem Mundspray, einer Inhalierungsvorrichtung oder einer oralen Dosisform, wie beispielsweise einer Bukkalpackung, einer Lutschtablette, wie beispielsweise einer sublingualen Lutschtablette, einer Tablette oder einem Pulver, enthalten ist.

11. Verfahren zur Herstellung einer Nikotin enthaltenden pharmazeutischen Zusammensetzung nach einem der Ansürcühe 1 bis 10, **dadurch gekennzeichnet, dass** die Cellulose einem Nikotindampf oder einer alkoholischen Lösung von Nikotin oder Nikotinsalz ausgesetzt wird.

12. Verfahren zum Erzielen einer verringerten Verwendung von Tabak enthaltendem Material und/oder zum Vorsehen des Gefühls einer Rauchbefriedigung ohne Rauchen oder zum Erreichen einer Gewichtskontrolle, umfassend die Verabreichung einer Nikotin enthaltenden pharmazeutischen Zusammensetzung nach einem der Ansprüche 1 bis 10 an eine Person.

13. Verfahren zum Erreichen einer verringerten Verwendung von Tabak enthaltendem Material und/oder zum Vorsehen eines Gefühls einer Rauchbefriedigung ohne Rauchen oder zum Erreichen einer Gewichtskontrolle, wobei ein Verfahren nach Anspruch 12 in Kombination mit einem oder mehreren anderen Verfahren zum Erreichen einer verringerten Verwendung von Tabak enthaltendem Material und/oder zum Vorsehen des Gefühls einer Rauchbefriedigung ohne Rauchen oder zum Erreichen einer Gewichtskontrolle verwendet wird.

14. Nikotin enthaltende pharmazeutische Zusammensetzung nach einem der Ansprüche 1 bis 10 zur Verwendung in einer Therapie, bei der es sich um die Behandlung einer Krankheit, die aus der Gruppe bestehend aus einer Tabak- oder Nikotinabhängigkeit, Alzheimer-Krankheit, Morbus Crohn, Parkinson-Krankheit, Tourette-Syndrom, ulcerative Colitis ausgewählt ist; und die Gewichtskontrolle handelt.

15. Verwendung von Nikotin und Cellulose von Algen, Bakterien und/oder Pilzen zur Herstellung einer Nikotin enthaltenden pharmazeutischen Zusammensetzung nach einem der Ansprüche 1 bis 10 zur Behandlung einer Krankheit, die aus der Gruppe bestehend aus Tabak- oder Nikotinabhängigkeit, Alzheimer-Krankheit, Morbus Crohn, Parkinson-Krankheit, Tourette-Syndrom, ulverative Colitis ausgewählt ist, und zur Gewichtskontrolle.

**Revendications**

1. Composition pharmaceutique contenant de la nicotine, **caractérisée en ce qu'**il comprend de la nicotine absorbée de manière réversible dans et/ou adsorbée sur la cellulose obtenue à partir d'algues, de bactéries et/ou de champignons.

2. Composition pharmaceutique contenant de la nicotine selon la revendication 1, **caractérisée en ce qu'**au moins une partie de la cellulose est obtenue à partir d'une ou plusieurs algues choisies parmi
   les algues bleu-vert (Cyanophyta), comme l'Anabaena et la Nostoc punctiformae ;
   les algues vertes (Chlorophyta), comme la Chladophora glomerata et rupestris, la Chaetomorpha melagonium, l'Enteromorpha, l'Ulva lactuca, l'Oocystis, par exemple solitaria et apiculata, et la Valonia, par exemple ventricosa, et la Chara corallina ;
   les algues dorées (Chrysophyta), comme la Vaucheria ;
   les dinoflagellates (Pyrrophyta), comme la Crypthecodinium cohnii, la Gonyaulax polyedra, la Scrippsiella hexapraecingula, la Dinobryon et la Peridinium ;
   les algues marron (Phaeophyta), comme la Lessonia nigrescens, la Macrocystis pyrifera, l'Ascophylum nodosium, le Fucus serratus, l'Halidrys siliquosa, l'Himanthalia lorea, la Pelvetia canalicuta, la Laminaria digitata, et
   les algues rouges (Rhodophyta), comme la Griffithsia flusculosa, la Porphyra, la Ptilota plumosa, la Rhodymenia palmate et l'Erythrocladia subintegra.

3. Composition pharmaceutique contenant de la nicotine selon la revendication 1, **caractérisée en ce qu'**au moins une partie de la cellulose est obtenue à partir de bactéries choisies parmi les Acetobacter, comme les xylinum, pasteurianus, aceti et acetigenus ;
   Achromobacter ;
   Aerobacter ;
   Agrobacterium, comme l'Agrobacterium tumefaciens ;
   Alcaligenes ;
   Polyspondium pallidum ;
   Pseudomonas ;
   Rhizobium, comme le Rhizobium leguminosarum ;
   Sarcina ;
   Sphareofilus natans ;
   Xanthomonas campestris ; et
   Zoogloega.

**4.** Composition pharmaceutique contenant de la nicotine selon la revendication 1, **caractérisée en ce qu'**au moins une partie de la cellulose est obtenue à partir de champignons choisis parmi

Achlya bisexualis ;

Saprolegnia, comme parasitica et monoica ;

Dictyostelium, comme le Dictyostelium discoideum ;

Phytophtora, comme le parasitica var. nicotianae et cactorum ;

Microdochium nivale ;

Ophiostoma ulmi ;

Colletotrichum lindemuthianum, et

Pythium, comme l'aphanidermatum, butleri et ultimatum.

**5.** Composition pharmaceutique contenant de la nicotine selon l'une quelconque des revendications 1 à 4, **caractérisée en ce que** la nicotine est choisie à partir de sels de nicotine, de préférence tartrate, tartrate d'hydrogène, chlorhydrate, acétate ou salicylate, la forme de base libre de nicotine, la nicotine dans n'importe quelle liaison non covalente, et des mélanges de ceux-ci.

**6.** Composition pharmaceutique contenant de la nicotine selon l'une quelconque des revendications 1 à 5, **caractérisée en ce qu'**il comprend en outre un ou plusieurs agents aromatisants et/ou arômes, comme ceux choisis dans le groupe consistant en huiles essentielles obtenues par distillations, extractions par solvant ou expressions à froid de fleurs fraiches ou séchées, bourgeons, feuilles, tiges, fruits, graines, peau, écorce ou racine, par exemple huile de menthe poivrée, menthe verte, eucalyptus, wintergreen, niaouli, clou de girofle, cardamome, cannelle, amande amère, coriandre, carvi, gingembre, genévrier, orange, orange amère, citron, pamplemousse, mandarine, bergamote, thym, fenouil et romarin ; aromes naturels et agents aromatiques comprenant soit des solutions diluées de, par exemple, huiles essentielles ou concentrés de composants aromatiques d'origine naturelle à partir, par exemple, de fruits, de baies, de noix, d'épices, de menthes, de tabac, de cacao, de café, de thé, de vanille, de réglisse, de caramel, de caramel anglais, de miel, de vin, de liqueurs et de bières, et des mélanges de ceux-ci.

**7.** Composition pharmaceutique contenant de la nicotine selon l'une quelconque des revendications 1 à 6, **caractérisée en ce qu'**elle comprend en outre un ou plusieurs additifs stabilisants, comme ceux choisis dans le groupe constitué d'antioxydants comprenant la vitamine E, à savoir des tocophérols, la vitamine C, à savoir de l'acide ascorbique et ses sels, du pyrosulfite de sodium, du butylhydroxytoluène, de l'hydroxyanisole butylé ; et des conservateurs comprenant des parabènes, du chlorure de benzalkonium, du chlorobutanol, de l'alcool de benzyle, de l'alcool de bêtaphényléthyle, du chlorure de cétylpyridinium, de l'acide citrique, de l'acide tartrique, de l'acide lactique, de l'acide malique, de l'acide acétique, de l'acide benzoïque, et de l'acide sorbique et leurs sels ; et des agents chélatants, comme l'EDTA ; et des galates, comme le propyl-galate.

**8.** Composition pharmaceutique contenant de la nicotine selon l'une quelconque des revendications 1 à 7, **caractérisée en ce qu'**elle comprend en outre un ou plusieurs additifs choisis dans le groupe constitué de :

agents épaississants, comme des polymères naturels, semi-synthétiques ou synthétiques, par exemple de l'amidon et des dérivés d'amidon, de la cellulose et des dérivés de cellulose, des polyéthylène glycols et des dérivés de ceux-ci, des polyacrylates et des polyvinyl esters et éthers ;

des amplificateurs, comme l'azone ;

des vitamines comme les vitamines B, C et E ;

des minéraux, comme les fluorures, en particulier du fluorure de sodium, du monofluoro-phosphate de sodium et du fluorure d'étain ;

des anti-odeurs, comme le zinc et les cyclodextrines ;

des propulseurs, comme le 1,1,2,2-tétrafluoroéthane (HFC-134a), éventuellement liquéfié, et du 1,1,1,2,3,3,3-heptafluoropropane (HFC-227), éventuellement liquéfié ;

des édulcorants comprenant un ou plusieurs agents édulcorants synthétiques et/ou sucres naturels comme ceux choisis dans les groupes constitués d'édulcorants artificiels, par exemple la saccharine et ses sels de sodium et calcium ;

l'aspartame, l'acésulfame et son sel de potassium, la thaumatine, la glycyrrhizine, le sucralose, la dihydro-chalcone, l'alitame, la miraculine, la monelline et le stevside ;

les alcools polyhydriques comme le sorbitol, le xylitol, le mannitol et le glycérol ;

les monosaccharides comprenant le glucose également appelé dextrose, le fructose, également appelé lévulose, et le galactose ;

des disaccharides comprenant du saccharose, également appelé sucrose, le lactose également appelé sucre du lait, et le maltose, également appelé sucre du malt ;

des mélanges de sucres comprenant du sirop de glucose liquide, par exemple des hydrolysats d'amidon contenant principalement un mélange de dextrose, de maltose, de dextrines et d'eau, du sirop de sucre inverti, par exemple de sucrose inverti par invertase contenant un mélange de dextrose, de lévulose et d'eau, des sirops à teneur en sucre élevée comme la mélasse, le miel et l'extrait de malt ; et des mélanges de ceux-ci.

9. Composition pharmaceutique contenant de la nicotine selon l'une quelconque des revendications 1 à 8, **caractérisée en ce qu'**elle est alcalinisée par tamponnage et/ou régulation de pH en utilisant un ou plusieurs agents tampons choisis dans le groupe constitué d'un carbonate, comme le mono-carbonate, le bicarbonate et le sesquicarbonate ; le glycinate, le phosphate, le glycérolphosphate, l'acétate, le gluconate ou le citrate d'un métal alcalin, comme le potassium ou le sodium, ou d'ammonium, et des mélanges de ceux-ci ; et/ou en utilisant des agents régulateurs de pH, comme des agents choisis dans le groupe constitué d'hydroxyde de sodium, d'hydroxyde de potassium, d'hydroxyde de calcium et d'oxyde de calcium ; et/ou en utilisant au moins en partie des formes de régulation de pH de nicotine.

10. Composition pharmaceutique contenant de la nicotine selon l'une quelconque des revendications 1 à 9, **caractérisée en ce qu'**elle est comprise dans un dispositif d'inhalation, un patch transdermique, un spray nasal, un spray buccal, un dispositif d'inhalation, ou une forme de dosage orale, comme un sachet buccal, une pastille, comme une pastille sublinguale, un comprimé ou une poudre.

11. Procédé de préparation d'une composition pharmaceutique contenant de la nicotine selon l'une quelconque des revendications 1 à 10, **caractérisé en ce que** la cellulose est exposée à la vapeur de nicotine ou à une solution alcoolique de nicotine ou de sel de nicotine.

12. Procédé destiné à réduire l'utilisation de matériau contenant du tabac et/ou à fournir une sensation de satisfaction liée à l'action de fumer, sans fumer, ou pour atteindre un contrôle du poids, comprenant l'administration à un sujet d'une composition pharmaceutique contenant de la nicotine selon l'une quelconque des revendications 1 à 10.

13. Procédé destiné à réduire l'utilisation de matériau contenant du tabac et/ou à fournir une sensation de satisfaction liée à l'action de fumer, sans fumer, ou pour atteindre un contrôle du poids, dans lequel un procédé selon la revendication 12 est utilisé en combinaison avec un ou plusieurs autres procédés afin d'obtenir la réduction de l'utilisation d'un matériau contenant du tabac, et/ou de fournir une sensation de satisfaction liée à l'action de fumer, sans fumer, ou pour atteindre un contrôle du poids.

14. Composition pharmaceutique contenant de la nicotine selon l'une quelconque des revendications 1 à 10, à utiliser lors d'une thérapie, étant destinée au traitement d'une maladie sélectionnée dans le groupe constitué de l'addiction au tabac ou à la nicotine, la maladie d'Alzheimer, la maladie de Crohn, la maladie de Parkinson, le syndrome de Tourette, la rectocolite hémorragique, et le contrôle du poids.

15. Utilisation de nicotine et de cellulose provenant d'algues, de bactéries et/ou de champignons, pour la fabrication d'une composition pharmaceutique contenant de la nicotine, selon l'une quelconque des revendications 1 à 10, pour le traitement d'une maladie sélectionnée dans le groupe constitué de l'addition au tabac ou à la nicotine, la maladie d'Alzheimer, la maladie de Crohn, la maladie de Parkinson, le syndrome de Tourette, la rectocolite hémorragique, et le contrôle du poids.

## REFERENCES CITED IN THE DESCRIPTION

**Patent documents cited in the description**

- US 5810018 A
- US 5939100 A
- US 4967773 A
- US 4579858 A
- DE 3241437
- WO 9312764 A

- US 5167242 A
- US 6024097 A
- US 20020059939 A
- US 5254346 A
- DE 1517264

**Non-patent literature cited in the description**

- **RUSSELL et al.** *British Medical Journal,* 1983, vol. 286, 683
- **JARVIS et al.** *Brit. J. of Addiction,* 1987, vol. 82, 983
- **ROSE.** Pharmacologic Treatment of Tobacco Dependence. Harvard Univ. Press, 1986, 158-166
- Chemical Engineers' Handbook. Theory of sorption. McGrawHill Book Company, 1963, 16-4
- **SEGAL, L. ; CREELY, J. J. ; MARTIN, A. E. JR. ; CONRAD, C. M.** An empirical method for estimating the degree of crystallinity of native cellulose using the X-ray diffractometer. *Textile Res. J.,* October 1959, 786-794
- Cellulose and Cellulose Derivatives. Interscience Publishers Inc, 1943, 11

- **MATSUMOTO K. ; NAKAI Y. ; YAMEMOCHI E. ; OGUCHI T. ; YAMAMOTO K.** Effect of pore size on the gaseous adsorption of ethenzamid on porous crystalline cellulose and the physicochemical stability of ethenzamide after storage. *Chem. Pharm. Bull.,* 1998, vol. 46, 314-318
- **EK R ; GUSTAFSSON C ; NUTT A ; IVERSEN T ; NYSTRÖM C.** Cellulose powder from Cladophora species algae. *J. Mol. Recog.,* 1998, vol. 11, 263-265
- **KERGER DR ; CELL WALLS.** The physiology and biochemistry of algae. Academic press, 1962
- **KLEMM D ; SCHUMANN D ; UDHARDT U ; MARSCH S.** Bacterial synthesized cellulose - artificial blood vessels for microsurgery. *Prog.polym.Sci.,* 2001, vol. 26, 1561-1603